# EUROPÄISCHE PATENTSCHRIFT

(11) **EP 0 585 503 B1**
(45) Veröffentlichungstag und Bekanntmachung des Hinweises auf die Patenterteilung: **07.01.1998**
(21) Anmeldenummer: 92810668.1
(22) Anmeldetag: 01.09.1992
(51) Int. Cl.: A61F 2/34

(54) **Doppelschalige Revisionshüftgelenkpfanne**
Replacement hip acetabular cup with a double shell
Coque acétabulaire de remplacement pour la hanche à cupule double

(43) Veröffentlichungstag der Anmeldung: 09.03.1994
(73) Patentinhaber: Sulzer Orthopädie AG, 6340 Baar (CH)
(72) Erfinder: Willert, Hans-Georg, Prof. Dr. med., W-3400 Göttingen (DE); Koch, Rudolf, CH-8500 Frauenfeld (CH); Bider, Kurt, CH-8406 Winterthur (CH)
(74) Vertreter: Trieblnig, Adolf

(56) Entgegenhaltungen:
- EP-A- 0 091 315
- EP-A- 0 245 527
- EP-A- 0 303 006
- EP-A- 0 313 762
- EP-A- 0 341 199
- FR-A- 2 617 040

## Beschreibung

Die Erfindung handelt von einer doppelschaligen Revisionshüftgelenkpfanne bestehend aus einer metallischen Aussenschale, die mit Knochenschrauben an Durchbrüchen im Beckenknochen verankerbar ist, und aus einer intraoperativ einsetzbaren Innenschale, welche eine sphärische Lagerschale für einen Kugelkopf aufweist und mit einer Schnappverbindung an der Aussenschale einrastet.

Eine doppelschalige Hüftgelenkschale mit Schnappverbindung ist in der EP 0 313 762 gezeigt. Die Revision d.h. der Ersatz einer Hüftgelenkpfanne ist praktisch immer mit einem Substanzverlust am Beckenknochen verbunden. Der Operateur muss das Knochenbett nachbearbeiten und kommt zwangsläufig zu einer Abstützfläche, die in den Knochen hineinversetzt ist und ein grösseres Volumen beansprucht. Gleichzeitig sollte die sphärische Lagerschale für den Kugelkopf, bei optimaler Verankerung der Aussenschale, ihre Ideallage für den Kugelkopf beibehalten. Diesem Umstand trägt die Erfindung Rechnung. Sie hat die Aufgabe, dem Operateur mehr Freiheit für die Befestigung der Aussenschale zu geben, ohne die Aufnahmebedingungen an der sphärischen Lagerschale für einen Kugelkopf zu verschlechtern.

Diese Aufgabe wird durch die Kennzeichen des unabhängigen Anspruchs 1 gelöst. Die Vorteile der Erfindung sind darin zu sehen, dass mit der Ovalform der Aussenschale eine drehsichere Verbindung zum Beckenknochen und mit der Ovalform der Innenschale eine drehsichere Verbindung zur Aussenschale erreicht wird, die es gestattet Kippmomente abzufangen, wie sie entstehen, wenn das Zentrum der sphärischen Lagerschale vom Zentrum der Aussenschale gegen aussen versetzt ist. Weitere Vorteile ergeben sich durch die Kennzeichen der abhängigen Ansprüche 2 bis 5. Durch die Verwendung einer Kunststoffinnenschale entsteht eine Dämpfungswirkung bei stossartigen Belastungen. Im weiteren gestattet es der Kunststoff, Innenschalen bezüglich ihrer Befestigung an der Aussenschale vorzubearbeiten und als Halbfabrikate an Lager zu legen, um sie für extreme Lagen der sphärischen Lagerfläche kurzfristig fertig zu bearbeiten. Die geraden Zwischenstücke am Umfang und die eindeutigen Abstützflächen erleichtern das Spannen und Fertigbearbeiten der Halbfabrikate.

Im folgenden ist die Erfindung anhand von Ausführungsbeispielen beschrieben. Es zeigen:
- Fig. 1: schematisch die Ansicht auf eine fertig bearbeitete Innenschale mit sphärischer Lagerschale und mit einseitig überhöhtem Rand;
- Fig. 2: schematisch die Ansicht auf die Innenseite einer Aussenschale;
- Fig. 3: schematisch die Ansicht auf die Aussenseite einer Aussenschale; und
- Fig. 4: schematisch den Längsschnitt durch eine Innenschale, die eine stark lateral versetzte sphärische Lagerschale aufweist.

In den Figuren ist eine doppelschalige Hüftgelenkpfanne mit ovalem Querschnitt und sphärischer Lagerschale 4 gezeigt. Die metallische Aussenschale 1 weist konstante Wandstärke auf und ist mit Durchbrüchen 3 zur Verankerung im Beckenknochen versehen. Die Innenschale 2 aus Kunststoff ist durch Schwenken um einen Nocken 15 am Äquator der Aussenschale 1 und über federnde Zungen 11 an der gegenüberliegenden Seite mit der Aussenschale 1 verklinkbar. Die Ovalform ist durch Einsetzen eines geraden Zwischenstücks 5, 6 an Innen- und Aussenschale erzeugt. Durch die längliche Form der Aussenschale 1, die in unterschiedlichen Richtungen einsetzbar ist, kann eine optimale Befestigung angestrebt werden. Die definitive Lage der sphärischen Lagerschale 4 wird erst mit dem Einsetzen einer passenden Innenschale 2 festgelegt. Dabei können die sphärischen Lagerschalen 4 in der äquatorialen Ebene der Aussenschale 1 und/oder in Richtung der Polachse der Aussenschale verschoben sein.

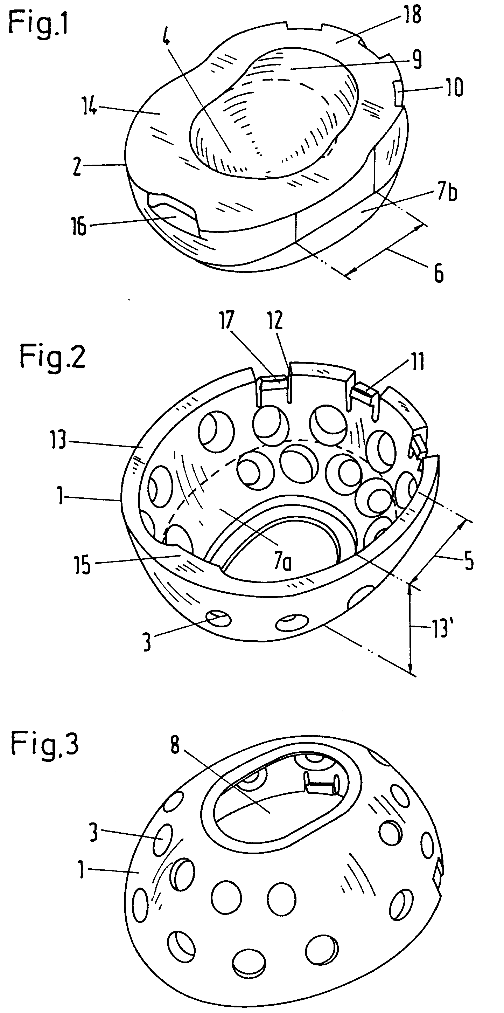

In Figur 1 ist die Innenschale 2 und darunter in Figur 2 die aufnehmende Aussenschale 1 der Hüftgelenkpfanne zu sehen. Innenschale 2 und Aussenschale 1 weisen in ihrem Querschnitt durch Einsetzen eines geraden Zwischenstücks 6 resp. 5 eine Ovalform wie ein Stadion auf. Diese Ovalform ist auch in den Abstützflächen zwischen Aussenschale und Innenschale fortgesetzt, welche aus umlaufenden Schrägflächen 7a resp. 7b bestehen und gerade Mantellinien aufweisen. Die Schrägflächen 7a, 7b werden miteinander verpresst, indem die Innenschale 2 mit einer Ausnehmung 16 an einem Nocken 15 am Äquator der Aussenschale angesetzt und quer zur Längsachse um diesen geschwenkt wird, bis eine Schnappverbindung 17 am gegenüberliegenden Äquatorrand einrastet. Die Schnappverbindung 17 wird durch federnde Zungen 11 vorgenommen, die durch Schlitze 12 in der Wand der Aussenschale entstanden sind, wobei die Zungen 11 in den Innenraum hineinragende schräge Auflaufflächen aufweisen, über welche die Zungen 11 beim Einschwenken der Innenschale vorgespannt werden, bis sie beim gegenseitigen Aufliegen der Schrägflächen 7a, 7b einrasten können.

Die Figur 3 zeigt im Polbereich der Aussenschale 1 einen ebenfalls ovalen Durchbruch 8, der beim Befestigen der Aussenschale als Kontrollöffnung dient und das Stopfen mit Knochenspänen erlaubt. Die eigentliche Befestigung der Aussenschale 1 geschieht mit Knochenschrauben (hier nicht gezeigt), die in angesenkten Durchbrüchen 3 mit ihren Köpfen aufliegen, um die gegenseitige Zentrierung der Schrägflächen 7a, 7b nicht zu behindern. Dabei sind die Durchbrüche 3 hauptsächlich auf die runden Bereiche der Aussenschale 1 in unterschiedlicher Höhe verteilt und gestatten auch im caudalen Bereich eine sichere Befestigung, um Momente über den als Hebelarm wirkenden mittleren Bereich mit dem geraden Zwischenstück 5 aufzunehmen.

In die bereits befestigte Aussenschale 1 sind Innenschalen 2 mit unterschiedlichen Lagen der sphärischen Lagerschale 4 zur Aussenschale 1 einsetzbar. Im weiteren kann der Radius der sphärischen Lagerschalen unterschiedlich sein. Durch die ovale Form der Aussenschale 1 können die sphärischen Lagerschalen 4 recht unterschiedliche Lage zur Aussenschale 1 einnehmen. So zeigt Figur 1 eine Innenschale 2, deren sphärische Lagerschale 4 mit dem Rand der Aussenschale abschliesst und an die eine Ueberhöhung 18 mit zylindrischer Führungsfläche 9 angesetzt ist, um Luxationen einer eingelegten Gelenkkugel zu verhindern.

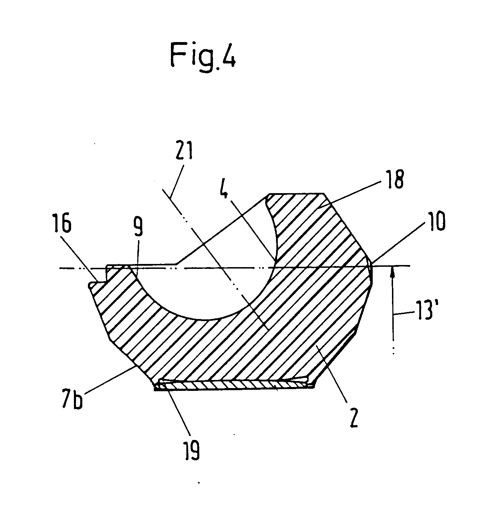

Figur 4 zeigt eine stark lateralisierte Lage der sphärischen Lagerschale 4. Der Radiusmittelpunkt der sphärischen Lagerschale 4 liegt weit ausserhalb der Randhöhe 13' der Aussenschale 1 und ihre Polachse 21 steht schräg zum Äquator der Aussenschale 1. Dabei ist die Polachse 21 dermassen geneigt, dass innerhalb der Randhöhe 13' der Aussenschale eine zylindrische Führungsfläche 9 entsteht.

Die Innenschale 2 der Figur 4 hat auf ihrer Unterseite eine ovale Ausnehmung 19, in die eine Metallplatte, vorzugsweise aus Titan, eingepresst ist. Mit dem Einsetzen der Innenschale 2 wird der Kunststoff der Innenschale 2 im Bereich vom Durchbruch 8 durch Metall gegenüber dem Knochenbett abgedeckt.

## Patentansprüche

1. Doppelschalige Revisionshüftgelenkpfanne bestehend aus einer metallischen Aussenschale (1), die mit Knochenschrauben an Durchbrüchen (3) im Beckenknochen verankerbar ist, und aus einer intraoperativ einsetzbaren Innenschale (2), welche eine sphärische Lagerschale (4) für einen Kugelkopf aufweist und mit einer Schnappverbindung (17) an der Aussenschale (1) einrastet, dadurch gekennzeichnet, dass die Aussenschale konstante Wandstärke besitzt, dass die Aussenschale (1) und die Innenschale (2) in ihrer Trennfläche einen ovalen Querschnitt aufweisen, indem am Umfang jeweils ein gerades Zwischenstück (5, 6) eingesetzt ist, dass eine passende Kunststoff-Innenschale durch Schwenken quer zur Längsachse um einen Nocken (15) der Aussenschale auf der dem Nocken gegenüberliegenden Seite an federnden Zungen (11) der Aussenschale (1) verklinkbar ist, und dass Innenschale (2) und Aussenschale (1) mit dem Verklinken auf einer am Schalengrund der Aussenschale (1) umlaufenden Schrägfläche (7a, 7b) miteinander verpresst sind, wobei die Schrägflächen (7a, 7b) gerade Mantellinien aufweisen.

2. Hüftgelenkpfanne nach Anspruch 1, dadurch gekennzeichnet, dass die Aussenschale (1) im Polbereich einen ovalen Durchbruch (8) als Kontrollöffnung sowie als Oeffnung für Knochen-Substanz-Hinterfütterung aufweist.

3. Hüftgelenkpfanne nach Anspruch 1 oder 2, dadurch gekennzeichnet, dass verschiedene Innenschalen (2), die sich durch unterschiedliche Lage der sphärischen Lagerschale (4) zur Aussenschale (1) und/oder durch unterschiedliche Kugel-Kalotten-Durchmesser unterscheiden, in die ovale Aussenschale (1) einsetzbar sind.

4. Hüftgelenkpfanne nach Anspruch 3, dadurch gekennzeichnet, dass der Rand (14) der Innenschale (2) eine einseitige Ueberhöhung (18) mit zylindrischer Führungsfläche (9) gegenüber der sphärischen Lagerschale (4) aufweist, um Luxationen einer eingelegten Gelenkkugel zu verhindern.

5. Hüftgelenkpfanne nach einem der Ansprüche 1 bis 4, dadurch gekennzeichnet, dass der Rand der sphärischen Lagerschale (4) über die Randhöhe (13') des Randes 13 der Aussenschale 1 vorsteht.

## Claims

1. A replacement hip acetabular fossa having a double shell and consisting of a metallic outer shell (1), which can be attached by bone screws to openings (3) in the pelvic bone, and of an inner shell (2) which can be inserted during the operation and which comprises a spherical bearing shell (4) for a spherical head and engages with a snap connection (17) on the outer shell (1),
**characterised in that** the outer shell possesses a constant wall thickness,
**in that** the outer shell (1) and the inner shell (2) comprise an oval cross section in their interface by a straight intermediate piece (5, 6) in each case being inserted at the periphery,
**in that** a matching plastic inner shell can be latched onto resilient tongues (11) of the outer shell (1) by swivelling transversally to the longitudinal axis around a cam (15) of the outer shell on the side opposite the cam,
**and in that** with the latching inner shell (2) and outer shell (1) are pressed together on a circumferential inclined face (7a, 7b) at the shell base of the outer shell (1), with the inclined faces (7a, 7b) having straight surface lines.

2. A hip acetabular fossa according to Claim 1,
**characterised in that** in the pole region the outer shell (1) comprises an oval opening (8) as an inspection hole and also as an aperture for lining with bony substance.

3. A hip acetabular fossa according to Claim 1 or 2,
**characterised in that** various inner shells (2), which differ by the varying position of the spherical bearing shell (4) to the outer shell (1) and/or by varying diameters of the spherical segment, can be inserted into the oval outer shell (1).

4. A hip acetabular fossa according to Claim 3,
**characterised in that** the edge (14) of the inner shell (2) comprises a unilateral raised portion (18) having a cylindrical guide face (9) opposite the spherical bearing shell (4) to prevent dislocations of an inserted articular sphere.

5. A hip acetabular fossa according to one of Claims 1 to 4,
**characterised in that** the edge of the spherical bearing shell (4) extends over the edge height (13') of the edge 13 of the outer shell 1.

## Revendications

1. Coque acétabulaire de remplacement pour la hanche à cupule double constituée d'une cupule extérieure métallique (1) qui peut être ancrée par des vis pour os à des perçages (3) dans l'os du bassin et d'une cupule intérieure (2) pouvant être mise en place de manière intra-opérative qui présente une coque de palier sphérique (4) pour une tête sphérique et qui s'enclenche par un assemblage par enclenchement (17) à la cupule extérieure (1), caractérisée en ce que la cupule extérieure a une épaisseur de paroi constante, en ce que la cupule extérieure (1) et la cupule intérieure (2) présentent à leur face de séparation une section transversale ovale, en ce qu'il est mis en place sur le pourtour respectivement une pièce intermédiaire rectiligne (5, 6), en ce qu'une cupule intérieure adaptée en matière synthétique, par un pivotement transversalement à l'axe longitudinal autour d'une came (15) de la cupule extérieure peut être enclenchée sur le côté opposé à la came à des languettes élastiques (11) de la cupule extérieure (1), et en ce que la cupule intérieure (2) et la cupule extérieure (1) sont assemblées par pression l'une avec l'autre par l'enclenchement sur une surface inclinée (7a, 7b) s'étendant au fond de la cupule extérieure (1), les surfaces inclinées (7a, 7b) présentant des génératrices rectilignes.

2. Coque acétabulaire selon la revendication 1, caractérisée en ce que la cupule extérieure (1) présente dans la zone polaire un perçage ovale (8) servant d'ouverture de contrôle et d'ouverture pour l'alimentation en substance osseuse.

3. Coque acétabulaire selon la revendication 1 ou 2, caractérisée en ce que diverses cupules intérieures (2) qui se différencient par une position différente de la coque de palier sphérique (4) relativement à la cupule extérieure (1) et/ou par des diamètres différents des calottes sphériques, peuvent être placées dans la cupule extérieure ovale (1).

4. Coque acétabulaire selon la revendication 3, caractérisée en ce que le bord (14) de la cupule intérieure (2) présente une surélévation unilatérale (18) avec une face de guidage cylindrique (9) relativement à la coque de palier sphérique (4) pour empêcher des luxations d'une sphère d'articulation mise en place.

5. Coque acétabulaire selon l'une des revendications 1 à 4, caractérisée en ce que le bord de la coque de palier sphérique (4) fait saillie sur la hauteur de bord (13') du bord (13) de la cupule extérieure (1).
